# DEMANDE DE BREVET EUROPEEN

(11) **EP 0 974 334 A1**
(43) Date de publication de la demande: **26.01.2000**
(21) Numéro de dépôt: 99401503.0
(22) Date de dépôt: 17.06.1999
(51) Int. Cl.: A61K 7/043, A61K 7/02, A61K 7/04, A61K 7/48

(54) **Composition de vernis pour le maguillage et/ou le soin des ongles contenant un composé fluoré volatile**

(30) Priorité: 30.06.1998 FR 9808340
(71) Demandeur: L'OREAL, 75008 Paris (FR)
(72) Inventeur: Bara, Isabelle, 75013 Paris (FR)
(74) Mandataire: Lhoste, Catherine

(57) **Abrégé**

Composition de vernis pour le maquillage et/ou le soin des ongles, incolore ou colorée, comprenant dans un système solvant organique pour vernis, une substance filmogène, un agent plastifiant, et éventuellement une résine. Ledit système solvant pour vernis contient au moins un composé halogéné volatil, dont l'halogène est le fluor, ayant une pression de vapeur supérieure à 20 mba (2000 Pa) à 25°C.

Cette composition est caractérisée par un séchage très rapide après application sur la surface des ongles.

## Description

La présente invention a pour objet une nouvelle composition cosmétique de vernis pour le maquillage et/ou le soin des ongles, incolore ou colorée, contenant en plus des ingrédients usuels, au moins un composé fluoré volatil.

Cette composition est caractérisée par un séchage très rapide après application sur la surface des ongles.

La présente invention a également pour objet un procédé de maquillage et/ou de soin des ongles.

La présente invention a en outre pour objet un procédé pour accélérer le temps de séchage d'une composition cosmétique de vernis.

A l'heure actuelle, les utilisatrices de vernis à ongles ont l'habitude d'appliquer de préférence le vernis en deux couches successives pour obtenir de bonnes qualités de tenue, de brillance et de résistance aux chocs mais, entre chaque application, il est nécessaire d'attendre au moins dix minutes avant d'obtenir un séchage complet du film aussi bien en surface qu'en profondeur.

Ainsi, depuis quelques années, les recherches se sont orientées vers la mise au point de vernis à ongles ayant des temps de séchage beaucoup plus courts en vue de répondre à une demande croissante des consommatrices.

Pour ce faire, différents polymères filmogènes ont été envisagés, mais ces derniers ne confèrent un séchage rapide qu'en surface.

Il a également été proposé d'utiliser certaines huiles volatiles, notamment des composés siliconés tels que les cyclodiméthicones ou les polydiméthylsiloxanes de faible poids moléculaire, ainsi que des produits hydrocarbonés tels que les isoparaffines et l'isododécane.

Toutefois, ces composés présentent un certain nombre d'inconvénients tels que notamment de faibles points éclairs, et pour certains une odeur persistante désagréable, limitant ainsi leur utilisation sur le plan cosmétique.

En plus de ce problème de rapidité de séchage, les compositions de vernis doivent conférer de bonnes propriétés filmogènes, le film une fois sec devant présenter un aspect homogène ainsi que des qualités de brillance et de résistance.

On a maintenant constaté après de nombreuses études sur divers types de composés qu'il était possible, en utilisant une certaine classe de composés fluorés volatils, de réduire de manière très significative le temps de séchage du film de vernis, tout en conservant une bonne homogénéité ainsi que des qualités de brillance et de résistance.

Par ailleurs, ces composés fluorés volatils sont des constituants de choix pour des compositions cosmétiques de vernis puisqu'ils sont inodores et incolores, contrairement à certains composés de l'art antérieur.

La présente invention a donc pour objet, à titre de produit industriel nouveau, une composition cosmétique de vernis pour le maquillage et/ou le soin des ongles, incolore ou colorée, contenant dans un système solvant organique pour vernis, une substance filmogène, éventuellement un agent plastifiant et éventuellement une résine, caractérisée par le fait que ledit système solvant pour vernis contient au moins un composé organique halogéné volatil dont l'halogène est le fluor, ayant une pression de vapeur supérieure à environ 20 mba (2000 Pa) à 25°C et de préférence supérieure à 40 mba.

L'invention a aussi pour objet l'utilisation d'un composé halogéné volatil dont l'halogène est le fluor, ayant une pression de vapeur supérieure à 20 mba (2000 Pa) à 25°C pour la préparation d'une composition de vernis à milieu solvant organique pour le maquillage et/ou le soin des ongles, ledit composé halogéné volatil étant destiné à en accélérer le temps de séchage.

Par composé organique halogéné, dont l'halogène est le fluor, on entend un composé organique ne comportant pas d'autres atomes d'halogène que des atomes de fluor.

Par solvant organique, on entend tout milieu liquide à température ambiante (25°C) non aqueux et en particulier à base de cétone, de glycol, d'ester, d'éther, d'alcane, de composé aromatique d'aldéhyde.

Parmi les composés organiques halogénés volatils, appelés aussi composés fluorés volatils, du système solvant des vernis selon l'invention et répondant au critère de pression de vapeur mentionné ci-dessus, on peut notamment citer :
1) les composés perfluorocycloalkyles répondant à la formule (I) suivante : dans laquelle : 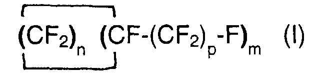
   n est 4 ou 5,
   m est 1 ou 2, et
   p est 1, 2 ou 3
   sous réserve que lorsque m = 2, les groupements ne sont pas nécessairement en alpha, l'un par rapport à l'autre ; et
2) les composés fluoroalkyles ou hétérofluoroalkyles répondant à la formule (II) suivante :

   CH₃-(CH₂)ₙ-[Z]ₜ-(CF₂)ₘ-CF₃ (II)

   dans laquelle :
   t est 0 ou 1,
   n est 0, 1, 2 ou 3,
   m est 2, 3, 4 ou 5, et
   Z est O, S ou NR, R étant hydrogène, un radical -(CH₂)ₙ-CH₃ ou -(CF₂)ₘ-CF₃.

Parmi les perfluorocycloalkyles de formule (I), on peut notamment citer le perfluorométhylcyclopentane et le perfluorodiméthylcyclohexane, vendus respectivement sous les dénominations de "Flutec PC1®" et "Flutec PC3®" par la Société BNFL FLUOROCHEMICALS Ltd.

Parmi les composés fluoroalkyles ou hétérofluoroalkyles de formule (II) on peut notamment citer le méthoxynonafluorobutane vendu sous la dénomination de "MSX 4518®" par la Société 3M (t=1, Z=O, n=0 et m=3) ou l'éthoxynonafluorobutane vendu sous la dénomination de "HFE 7200" par la Société Archimex (t=1, Z=O, n=1 et m=3).

Dans les compositions de vernis selon l'invention, le système solvant est généralement présent en une proportion comprise entre 50 et 90 % en poids par rapport au poids total du vernis et le composé fluoré volatil représente environ de 5 à 90 % et de préférence de 10 à 50 % du poids total du vernis.

Le temps de séchage des compositions de vernis étant fonction de la concentration en composé halogéné volatil, il est donc possible de faire varier, à partir d'une même composition de vernis, le temps de séchage.

Parmi les solvants organiques constituant le système solvant du vernis, on peut citer, en plus du composé fluoré volatil, l'acétone, l'acétate de méthyle, l'acétate d'éthyle, l'acétate de butyle, l'acétate d'amyle, l'acétate de méthoxy-2 éthyle, la méthyléthylcétone (MEC), la méthylisobutylcétone (MIC), l'acétate d'isopropyle, l'éthanol, l'isopropanol, l'éthylène glycol, le propylène glycol, le pentylène glycol, le glycérol, le décane, l'heptane, le cyclohexane, le benzaldéhyde, le diéthyléther, le diméthyléther, l'octane ou le toluène, le xylène, le n-butanol, le n-propanol, et leurs mélanges.

Selon une forme de réalisation particulière, le système solvant peut également contenir des silicones volatiles telles que :
1) les polydiméthylsiloxanes linéaires de faible poids moléculaire répondant à la formule (III) suivante : 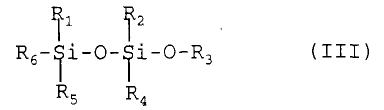 dans laquelle :
   R₁ à R₆, identiques ou différents, représentent un atome d'hydrogène, un hydroxyle, un radical alkyle ou alkényle en C₁-C₆ ;
2) les polydiméthylsiloxanes cycliques répondant à la formule (IV) suivante : 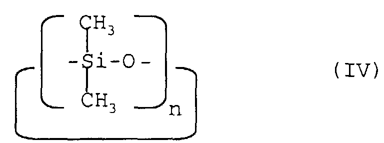 dans laquelle n est 4, 5 ou 6.

Parmi les composés de formule (IV), on peut notamment citer :
- l'octaméthylcyclotétrasiloxane,
- le décaméthylcyclopentasiloxane, et
- le dodécaméthylcyclohexasiloxane.

La substance filmogène des vernis selon l'invention est généralement présente en une proportion d'environ 5 à 20 % et de préférence d'environ 10 à 20 % en poids par rapport au poids total de la composition de vernis.

Parmi ces substances filmogènes, on peut notamment citer les nitrocelluloses du type "RS" ou "SS" et en particulier la nitrocellulose type ¼ seconde RS, la nitrocellulose type ½ seconde RS, la nitrocellulose type ½ seconde SS et la nitrocellulose type ¾ seconde RS.

Comme autre substance filmogène, on peut également utiliser des polymères ou copolymères acryliques, des résines du type acrylique, styrénique, acrylate-styrénique et vinylique, des copolymères vinyliques, des polymères polyesters, et des polymères polyuréthannes.

L'agent plastifiant des vernis selon l'invention est généralement présent en une proportion comprise entre 2 et 15 %, et de préférence entre 5 et 10 % en poids par rapport au poids total de la composition de vernis.

Les agents plastifiants permettent de régler la flexibilité du film sans affaiblir sa résistance ou sa force physique.

Parmi les agents plastifiants susceptibles d'être utilisés dans les compositions cosmétiques de vernis selon l'invention, on peut citer: le phosphate de tributyle, le phosphate de tributoxy-éthyle, le phosphate de tricrésyle, le phosphate de triphényle, le triacétate de glycérol, le stéarate de butyle, le glycolate de butyle, le benzoate de benzyle, l'acétyl-ricinoléate de butyle, l'acétyl-ricinoléate de glycéryle, le phtalate de dibutyle, le phtalate de di-isobutyle, le phtalate de dioctyle, le phtalate de diméthoxyéthyle, le phtalate de diamyle, le citrate de triéthyle, le citrate de tributyle, l'acétyl-citrate de tributyle, l'acétyl-citrate de triéthyl-2 hexyle, le tartrate de dibutyle, le camphre, et leurs mélanges.

Les compositions de vernis selon l'invention peuvent également contenir une résine en une proportion comprise entre 0,5 et 15 %, et de préférence entre 5 et 10 % en poids par rapport au poids total des compositions.

Parmi les nombreuses résines utilisables, on préfère employer selon l'invention des résines du type aryl-sulfonamide formaldéhyde ou aryl-sulfonamide époxy, notamment la résine toluène sulfonamide formaldéhyde plus connue sous les dénominations commerciales de "SANTOLITE MHP®", "SANTOLITE MS 80 %®", et "KETJENFLEX MS 80®", ou encore des résines alkydes telles que celles vendues par la Société DAI NIPPON sous la dénomination de "BECKOSOL ODE 230-70®".

Ces résines, tout en augmentant le pouvoir filmogène, améliorent le brillant ainsi que l'adhérence.

Lorsque les compositions selon l'invention sont colorées, celles-ci peuvent contenir en outre des colorants et/ou pigments de nature organique ou inorganique.

Parmi les pigments organiques, on peut citer les D&C Red No.10, 11, 12 et 13, le D&C Red No. 7, les D&C Red No.5 et 6 et le D&C Red No.34, ainsi que les laques telles que la laque D&C Yellow No.5 et la laque D&C Red No.2.

Parmi les pigments inorganiques, on peut citer le dioxyde de titane, l'oxychlorure de bismuth, l'oxyde de fer brun et les oxydes de fer rouge.

D'une façon générale, les colorants et/ou pigments sont présents dans les compositions de vernis selon l'invention en une proportion comprise entre 0,01 et 2 % en poids par rapport au poids total des compositions.

En vue d'éviter la sédimentation des pigments, certains agents thixotropes tels les bentones peuvent être employés comme par exemple la "BENTONE 27®" ou la "BENTONE 38®".

Les compositions de vernis selon la présente invention peuvent également contenir des ingrédients et/ou actifs cosmétiques conventionnels, en particulier des composés actifs pour le traitement des ongles. Parmi ceux-ci, on peut citer les filtres UVA et/ou UVB, les agents dispersants et mouillants, les agents matifiants, les agents d'adhérence, les agents d'étalement, les agents rhéologiques tels que les silicones pyrogénées, les antioxydants, les conservateurs, les épaississants ou gélifiants et les agents durcisseurs des ongles.

La présente invention a également pour objet un procédé de préparation d'une composition de vernis à milieu solvant organique pour le maquillage et/ou le soin des ongles en vue d'en accélérer le temps de séchage, ce procédé consistant à introduire dans ladite composition une quantité efficace d'au moins un solvant halogéné volatil, dont l'halogène est le fluor, ayant une pression de vapeur supérieure à 20 mba (2000 Pa) à 25°C.

On va maintenant donner à titre d'illustration plusieurs exemples de compositions de vernis selon l'invention.

### EXEMPLES DE PREPARATION

### Exemple 1 : Vernis à ongles coloré

On prépare un vernis à ongles coloré en procédant au mélange des ingrédients suivants :
- Nitrocellulose à 30% dans l'alcool isopropylique 10 g
- Acétate d'éthyle 41 g
- Méthoxynonafluorobutane vendu sous la dénomination de MSX 4518 par la Société 3M 20 g
- Phtalate de dibutyle 15 g
- Agent gélifiant 1,5 g
- Pigments 1,5 g
- Résine toluène sulfonamide formaldéhyde 11 g

Ce vernis, de bonne fluidité, s'applique facilement sur la surface des ongles et conduit, après un temps de séchage très court, à la formation d'un film homogène ayant une bonne adhérence et un bel éclat.

### Exemple 2 : Vernis à ongles incolore

On prépare un vernis à ongles incolore en procédant au mélange des ingrédients suivants :
- Nitrocellulose à 30% dans l'alcool isopropylique 18,5 g
- Acétate d'éthyle 27,9 g
- Méthoxynonafluorobutane vendu sous la dénomination de MSX 4518 par la Société 3M 19,2 g
- Acétate de butyle 9,42 g
- Alcool isopropylique 6,28 g
- Acétylcitrate de tributyle 6,3 g
- Résine toluène sulfonamide formaldéhyde 11,00 g
- Camphre 1,4 g

## Revendications

1. Composition de vernis pour le maquillage et/ou le soin des ongles, incolore ou colorée, comprenant dans un système solvant organique pour vernis, une substance filmogène, éventuellement un agent plastifiant, et éventuellement une résine, caractérisée par le fait que ledit système solvant pour vernis contient au moins un composé organique halogéné volatil, dont l'halogène est le fluor, ayant une pression de vapeur supérieure à 20 mba (2000 Pa) à 25°C.

2. Composition selon la revendication 1, caractérisée par le fait que ledit composé halogéné volatil est un composé perfluorocycloalkyle répondant à la formule (I) suivante : 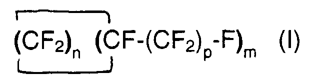 dans laquelle :
n est 4 ou 5,
m est 1 ou 2, et
p est 1, 2 ou 3
sous réserve que lorsque m = 2, les groupements ne sont pas nécessairement en alpha, l'un par rapport à l'autre.

3. Composition selon la revendication 2, caractérisée par le fait que le composé perfluorocycloalkyle est choisi dans le groupe constitué par le perfluorométhylcyclopentane et le perfluorodiméthylcyclohexane.

4. Composition selon la revendication 1, caractérisée par le fait que ledit composé halogéné volatil est un composé fluoroalkyle ou hétérofluoroalkyle répondant à la formule (II) suivante :
CH₃-(CH₂)ₙ-[Z]ₜ-(CF₂)ₘ-CF₃ (II)
dans laquelle :
t est 0 ou 1,
n est 0, 1, 2 ou 3,
m est 2, 3, 4 ou 5, et
Z est O, S ou NR, R étant hydrogène, un radical
-(CH₂)ₙ-CH₃, ou -(CF₂)ₘ-CF₃.

5. Composition selon la revendication 4, caractérisée par le fait que le composé hétérofluoroalkyle est le méthoxynona-fluorobutane ou l'éthoxynonafluorobutane.

6. Composition selon l'une quelconque des revendications précédentes, caractérisée par le fait que ledit système solvant est présent en une proportion comprise entre 50 et 90 % en poids par rapport au poids total de la composition.

7. Composition selon l'une quelconque des revendications précédentes, caractérisée par le fait que le composé halogéné volatil est présent en une proportion comprise entre 5 et 90 % en poids par rapport au poids total de la composition.

8. Composition selon l'une quelconque des revendications précédentes, caractérisée par le fait que le système solvant comprend au moins un solvant organique choisi parmi l'acétone, l'acétate de méthyle, l'acétate d'éthyle, l'acétate de butyle, l'acétate de méthoxy-2 éthyle, la méthyléthylcétone (MEC), la méthylisobutylcétone (MIC), l'acétate d'isopropyle, l'isopropanol, l'éthanol, l'éthylène glycol, le glycérol, le propylène glycol.

9. Composition selon l'une quelconque des revendications précédentes, caractérisée par le fait que le système solvant organique comprend en outre au moins une silicone volatile choisie parmi :
a) les polydiméthylsiloxanes linéaires de faible poids moléculaire répondant à la formule (III) suivante : 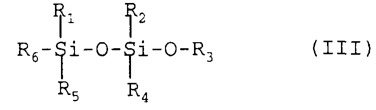 dans laquelle :
R₁ à R₆, identiques ou différents, représentent un atome d'hydrogène, un hydroxyle, un radical alkyle ou alkényle en C₁-C₆ ; et
b) les polydiméthylsiloxanes cycliques répondant à la formule (IV) suivante : 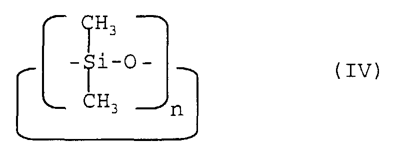 dans laquelle n est 4, 5 ou 6.

10. Composition selon la revendication 8, caractérisée par le fait que le composé de formule (IV) est choisi dans le groupe constitué par l'octaméthylcyclotétrasiloxane, le décaméthylcyclopentasiloxane, et le dodécaméthylcyclohexasiloxane.

11. Composition selon l'une quelconque des revendications précédentes, caractérisée par le fait que la substance filmogène est présente en une proportion comprise entre 5 et 20 % en poids par rapport au poids total de la composition.

12. Composition selon l'une quelconque des revendications précédentes, caractérisée par le fait que l'agent plastifiant est présent en une proportion comprise entre 2 et 15 % en poids par rapport au poids total de la composition.

13. Composition selon l'une quelconque des revendications précédentes, caractérisée par le fait que la résine est présente en une proportion comprise entre 0,5 et 15 % en poids par rapport au poids total de la composition.

14. Composition selon l'une quelconque des revendications précédentes, caractérisée par le fait que la composition comprend en outre au moins un pigment et/ou colorant présent en une proportion comprise entre 0,01 et 2 % en poids par rapport au poids total de la composition.

15. Composition selon l'une quelconque des revendications précédentes, caractérisée par le fait que la composition contient en outre au moins un ingrédient cosmétique usuel pour vernis ou au moins un composé actif de traitement des ongles.

16. Utilisation d'un composé halogéné volatil dont l'halogène est le fluor, ayant une pression de vapeur supérieure à 20 mba (2000 Pa) à 25°C pour la préparation d'une composition de vernis à milieu solvant organique pour le maquillage et/ou le soin des ongles, ledit composé halogéné volatil étant destiné à en accélérer le temps de séchage.

17. Procédé de préparation d'une composition de vernis à milieu solvant organique pour le maquillage et/ou le soin des ongles en vue d'en accélérer le temps de séchage, caractérisé par le fait qu'il consiste à introduire dans ladite composition au moins un solvant halogéné volatil, dont l'halogène est le fluor, ayant une pression de vapeur supérieure à 20 mba (2000 Pa) à 25°C.
